(19) Europäisches Patentamt
European Patent Office
Office européen des brevets

(11) Publication number: **0 193 454**
**A1**

(12) # EUROPEAN PATENT APPLICATION

(21) Application number: 86400334.8

(22) Date of filing: 18.02.86

(51) Int. Cl.[4]: C 07 D 473/18
A 61 K 31/52

(30) Priority: 25.02.85 US 704991

(43) Date of publication of application:
03.09.86 Bulletin 86/36

(84) Designated Contracting States:
CH DE FR GB IT LI NL

(71) Applicant: MERCK & CO. INC.
126, East Lincoln Avenue P.O. Box 2000
Rahway New Jersey 07065(US)

(72) Inventor: Ashton, Wallace T.
122 Sweet Briar Drive
Clark New Jersey 07066(US)

(72) Inventor: Tolman, Richard L.
29 Upper Warren Way
Warren New Jersey 07060(US)

(74) Representative: Ahner, Francis et al,
CABINET REGIMBEAU 26, avenue Kléber
F-75116 Paris(FR)

(54) **8-Substituted-9-hydroxyalkyl and hydroxyalkoxymethyl-guanines and pharmaceutical compositions containing them.**

(57) Novel 8-substituted-9-hydroxyalkyl- and hydroxyalkoxymethyl-guanines and pharmaceutically-acceptable salts thereof which are superior inhibitors of purine nucleoside phosphorylase (PNP) and which possess utility alone and in coadministration with other active drugs as immunosuppresive agents and in the treatment of cancer, especially of leukemia, of hyperuricemic states, and of virus infections.

Croydon Printing Company Ltd.

2756P/0836A



TITLE OF THE INVENTION

8-SUBSTITUTED-9-HYDROXYALKYL AND HYDROXYALKOXYMETHYL-GUANINES AND PHARMACEUTICAL COMPOSITIONS CONTAINING THEM.

The present invention is directed to novel 8-substituted-9-hydroxyalkyl- and hydroxyalkoxymethyl-guanines and pharmaceutically-acceptable salts thereof. These compounds are superior inhibitors of purine nucleoside phosphorylase (PNP) and possess utility in the treatment of cancer, especially of leukemia, as well as in the treatment of virus infections, such as herpes viruses.

BACKGROUND OF THE INVENTION

It has been suggested by Stoeckler *et al.*, *Biochemical Pharmacology*, *31*, No. 2, pp. 163-171, 1982, among others, that inhibition of the erythrocytic purine nucleoside phosphorylase (PNP) catalytic activity in mammals might give rise to immunosuppresive activity and might have significant

chemotherapeutic utility for the treatment of tumors and hyperuricemic states. It is suggested that in the absence of the salvage enzyme, PNP, either due to a natural deficiency or to potent inhibition, 2'-deoxyguanosine administered to a patient is converted to corresponding nucleotides and that accumulated dGTP reduces deoxynucleotide pools through feedback inhibition of ribonucleotide diphosphate reductase which may be toxic to cells undergoing DNA synthesis. Coadministration of a potent PNP-inhibitor may also permit certain potentially-effective chemotherapeutic purine nucleoside analogs, which compounds or their metabolic products are substrates for PNP, to survive bloodstream transport and reach their tumor targets before being converted in the vascular system to the respective free bases which may be less effective than the nucleosides. Finally, by facilitating the excretion of nucleic acid degradation products other than uric acid, PNP-inhibitors may have therapeutic utility in hyperuricemic states, such as gout and the neurologic disorder, Lesch-Nyhan syndrome.

Known inhibitors of PNP, such as 8-aminoguanine, 8-aminoguanosine, 5'-deoxy-5'-iodoinosine or 5'-chloro-5'-deoxyformycin B, are either substrates for PNP as well as being inhibitors of the enzyme, or have only moderate affinity for the enzyme. In order for substances to be useful as PNP-inhibitors, these materials must be poor enzyme substrates while still demonstrating high binding affinity for the enzyme, which would simulate the situation found in the PNP-immunodeficient state.

It was therefore an object of this invention to provide novel compounds having potency as purine nucleoside phosphorylase-inhibitors. It was another object of this invention to provide a chemotherapeutic method of treatment for cancers.

DESCRIPTION OF THE INVENTION

This present invention relates to novel 8-substituted-9-hydroxyalkyl- and hydroxyalkoxymethyl-guanines of the formula:

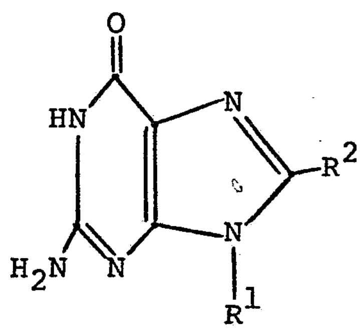

wherein:

$R^1$ is mono- or polyhydroxy-$C_1$-$C_6$-straight-or branched-chain alkyl, containing 1 to 5 hydroxyl groups, such as 2-hydroxyethyl, 3-hydroxypropyl, 4-hydroxybutyl, 5-hydroxypentyl, 6-hydroxyhexyl, 2,3-dihydroxypropyl, 2,4-dihydroxybutyl, 3,4-dihydroxybutyl, 2,3,4-trihydroxybutyl, 4-hydroxy-3-hydroxymethyl-1-butyl or 3-hydroxy-2-hydroxymethyl -1-propyl, or mono- or polyhydroxy-$C_1$-$C_6$-straight or branched-chain alkoxymethyl, containing 1 to 5 hydroxy groups, such as 1,3-dihydroxy-2-propoxymethyl, 2,3-dihydroxy-1-propoxymethyl, 2,4-dihydroxy-1-butoxymethyl,

(3-hydroxy-2-hydroxymethyl-1-propoxy)methyl or 1,3,4-trihydroxy-2-butoxymethyl, but not including $CH_2OCH_2CH_2OH$; and

$R^2$ is Cl, Br, F, I, $NH_2$, OH, SH, $R^3$, $SR^3$, $NHR^3$, $NR^3R^4$ or $OR^3$, where $R^3$ and $R^4$ are independently $C_1$-$C_{20}$ straight- or branched-chain, saturated or unsaturated (where the degree of unsaturation is limited to a maximum of 3 double bonds) alkyl (such as methyl, ethyl, propyl, isopropyl, hexyl, octyl, 2-octyl, dodecyl, hexadecyl, allyl or 2-buten-1-yl), which may or may not contain a $C_3$-$C_{10}$ mono- or bicyclic group, including aromatics or heteroaromatics (as in the case of benzyl, phenylethyl, furfuryl, 2-pyridylmethyl, 2-(2-pyridyl)ethyl, 2-(3-pyridyl)ethyl, 2-pyrimidinylmethyl, 2-(3-thienyl)ethyl, 2-(2-thiazolyl)ethyl, 2-(2-imidazolyl)ethyl, cyclohexylmethyl or 2-cyclopropylethyl); or $C_3$-$C_{10}$ mono- or bicyclic, saturated or unsaturated alkyl, aromatic or heteroaromatic, where the degree of unsaturation is limited to a maximum of 5 double bonds (such as cyclohexyl, cyclopropyl, phenyl, 2-naphthyl, 2-pyridyl, 3-pyridyl, 2-pyrimidinyl, 2-pyrazinyl, 3-pyrazolyl, 5-tetrazolyl, 1-indanyl, 2-decalinyl, 2-imidazolyl, 3-isoxazolyl, 2-oxazolyl, 8-quinolinyl, 2-benzimidazolyl, 2-thienyl, 3-thienyl, 2-thiazolyl, 4-thiazolyl, 2-benzthiazolyl, 1,3,5-triazin-2-yl, 1,2,4-triazol-3-yl or 1,3,4-thiadiazol-2-yl); or

where, in $NR^3R^4$, $R^3$ and $R^4$ together are a $C_3$-$C_{10}$ alkylene group (such that $NR^3R^4$ is, for example, piperidino or pyrrolidino);

and pharmaceutically-acceptable salts thereof.

The compounds of the instant invention include the racemic mixture of the stereo configurations as well as both the (R)- and (S)-enantiomers.

Examples of the compounds according to the present invention include:

8-amino-9-(1,3-dihydroxy-2-propoxymethyl)guanine;
8-amino-9-(2,3-dihydroxy-1-propoxymethyl)guanine;
8-amino-9-(4-hydroxybutyl)guanine;
8-amino-9-(3,4-dihydroxybutyl)guanine;
8-amino-9-[4-hydroxy-3-hydroxymethyl-1-butyl] guanine;
8-hydroxy-9-(1,3-dihydroxy-2-propoxymethyl)guanine;
8-methyl-9-(4-hydroxybutyl)guanine;
8-mercapto-9-(1,3-dihydroxy-2-propoxymethyl) guanine;
8-methylamino-9-(1,3-dihydroxy-2-propoxymethyl) guanine;
8-amino-9-(1,4-dihydroxy-2-butoxymethyl)guanine;
8-amino-9-[(3-hydroxy-2-hydroxymethyl-1-propoxy) methyl]guanine;
8-bromo-9-(1,3-dihydroxy-2-propoxymethyl)guanine;
8-benzyloxy-9-(1,3-dihydroxy-2-propoxymethyl) guanine; and
8-hydroxy-9-(4-hydroxybutyl)guanine.

Preferred compounds according to the instant invention are those wherein $R^1$ contains at least one primary hydroxyl group, such as $CH_2OCH(CH_2OH)_2$ or $CH_2OCH_2CHOHCH_2OH$ and $R^2$ is $NH_2$. Such compounds include 8-amino-9-(1,3-dihydroxy-2-propoxymethyl)guanine, 8-amino-9-(2,3-dihydroxy-1-propoxymethyl)guanine and pharmaceûtically-acceptable salts thereof.

These novel acyclonucleoside compounds, in addition to having antiviral activity, demonstrate very good PNP-binding affinities and low reaction rates.

The pharmaceutically-acceptable salts of the compounds of the instant invention include the conventional soluble, non-toxic salts of the compounds of this invention formed, for example, from non-toxic inorganic or organic acids. Such conventional non-toxic salts include those derived from inorganic acids, such as hydrochloric, hydrobromic, sulfuric, sulfamic, phosphoric or nitric, or from organic acids, such as ethane disulfonic, trifluoroacetic, isethionic, acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, and the like.

The present invention also relates to the preparation of these 8-substituted-9-hydroxyalkyl or hydroxyalkoxymethyl-guanines by three general synthetic methods. Method A consists of direct halogenation of the 9-hydroxyalkyl- or 9-hydroxyalkoxymethylguanine at the 8-position. This general method has been reviewed by J. H. Lister in "Fused Pyrimidines, Part II: Purines," ed by D. J. Brown [Vol. 24 (Part II) in "The Chemistry of Heterocyclic Compounds," A. Weissberger and E. C. Taylor, eds.], Wiley-Interscience, New York (1971), pp. 146-147. The 8-bromo derivatives, for example, are particularly well prepared by this method.

In Method B, a 9-hydroxyalkyl- or 9-hydroxyalkoxymethylguanine substituted at the 8-position by a leaving group, for example, an 8-bromo derivative prepared by Method A, is reacted with a nucleophile to introduce a new substituent at the 8-position. Thus, the 8-bromo group can be replaced in this way by alkoxide, phenoxide, mercaptide, substituted amino, and other related groups, These general reactions have been reviewed by Lister (ref. cited above, pp. 158-186). Unsubstituted 8-amino-9-hydroxyalkyl- or -hydroxyalkoxymethyl-guanines are readily prepared by reaction of the corresponding 8-bromoguanine (from Method A) with hydrazine, as the intermediate 8-hydrazino compound breaks down to give the 8-amino derivative [Lister, ref. cited above, pp. 169-170; R.E. Holmes and R. K. Robins, J. Am. Chem. Soc., 87, 1772 (1965)]. The 8-hydroxy-9-hydroxyalkyl-or-hydroxyalkoxymethylguanines may also be obtained using Method B by initial displacement of a bromo group (or other leaving group) at the 8-position by benzyloxide, followed by a deprotection step in which the O-benzyl group is removed by hydrogenolysis or other methods known in the literature [R. E. Holmes and R. K. Robins, J. Am. Chem. Soc., 87, 1772 (1965)].

Method C consists of ring-closure reaction of a 5-amino-6-(hydroxyalkylamino)isocytosine with an appropriate "one-carbon reagent" to give an 8-substituted-9-(hydroxyalkyl)guanine. The 5-amino-6-(hydroxyalkylamino)isocytosine may be prepared from a 6-hydroxyalkylamino-5-nitroso- or 5-nitroisocytosine [see, for example, A. Yamazaki, Chem. Pharm. Bull., 17, 1268 (1969)] by reduction using any of several known methods, such as catalytic hydrogenation, zinc

dust, or sodium dithionite. The resulting 5,6-diaminoisocytosine may be reacted with a variety of reagents to give 8-substituted guanines. For example, fusion with urea gives an 8-hydroxyguanine, and fusion with thiourea gives an 8-mercaptoguanine, which may then be alkylated on sulfur. Reaction of the diaminoisocytosine with an organic acid derivative under dehydrative conditions furnishes the 8-alkyl- or 8-arylguanine. The 5-amino group may be acylated prior to ring closure. Thus, heating a 5-acetamido-6-(hydroxyalkylamino)isocytosine with acetic anhydride gives the 9-hydroxyalkyl-8-methylguanine. These and related ring-closure reactions of 5,6-diaminopyrimidines with "one-carbon reagents" to give 8-substituted purines have been well reviewed by Lister (op. cit., pp. 31-90).

A pharmaceutical composition may then be prepared comprising an effective amount of a compound according to the present invention or a pharmaceutically-acceptable salt thereof together with a pharmaceutically-acceptable carrier therefor, such as aseptic saline solution, lactose or corn starch, with or without an adjuvant, such as alum. Useful pharmaceutical compositions according to the present invention may also contain other active ingredients, such as 6-mercaptopurine, 6-thioinosine or 6-thioguanosine, which are potentiated by the inclusion of a PNP-inhibitor, in effective amounts which may be coadministered with the compound or pharmaceutically-acceptable salt thereof according to the present invention.

These pharmaceutical compositions may be given orally or parenterally to mammalian, avian or piscine species, at dose levels of from about 0.1 to about 250 mg per kg, preferably from about 1.0 to 50 mg per kg, of animal body weight, and are used in humans in a unit dosage form which is administered as needed in an amount of about 1 to about 250 mg per dose.

For oral administration, fine powders or granules of the compositions according to the invention may contain diluting, dispersing and/or surface active agents, and may be presented in a draught, in water or in a syrup; in capsules or sachets in the dry state, or in a non-aqueous solution or suspension, wherein suspending agents may be included; in tablets, wherein binders and lubricants may be included; or in a suspension in water or a syrup. Where desirable or necessary, flavoring, preserving, suspending, thickening or emulsifying agents may be included. Tablets and granules are preferred, and these may be coated.

For parenteral administration or for administration as drops, the compounds may be presented in aqueous solution in a concentration of from about 0.1 to 10%, more preferably 0.1 to 7%, most preferably 0.2% weight/volume. The solution may contain antioxidants, buffers, and/or other known additives.

The invention is further defined by reference to the following examples, which are intended to be illustrative and not limiting.

## EXAMPLE 1

### Preparation of 8-Bromo-9-(1,3-dihydroxy-2-propoxymethyl)guanine

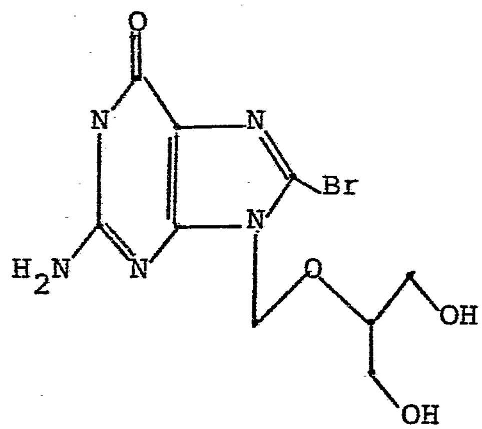

The compound, 9-(1,3-dihydroxy-2-propoxymethyl)guanine (10.00 g, 39.2 mmole) prepared as shown in Ashton et al., Biochem. Biophysics Res. Comm., 108, 1716-1721 (1982), was suspended in water (500 ml) at 22°C, and with vigorous stirring, a solution of bromine (3.0 ml, 58.2 mmole) in ice water (300 ml) was added dropwise to the suspension over 30 minutes. After the addition of approximately one-third of the bromine solution, the mixture became almost homogeneous, then became progressively an increasingly thick crystalline slurry.

The orange-colored mixture was stirred for an additional twenty minutes, then filtered.

The solid product was washed with water (2 X 50 ml); then with acetone (50 ml); then dried at 22°C *in vacuo*; and finally recovered as colorless micro needles (10.65 g), 81%, m.p.> 300°C. $R_f$ 0.55 (silica, $CHCl_3$; aq. 90% MeOH 60:40). pmr (200 MHz) ($D_2O$) δ 3.62 (d ABq, 4H, J=12, 6 and 4 Hz, ($-CH_2O-)_2$), 3.85 (m, 1H), 5.63 (s, 2H, $NCH_2O$).

Anal. for $C_9H_{12}BrN_5O_4 \cdot 1.1\ H_2O$:
Calc'd: C, 31.99; H, 4.24; N, 20.72; Br, 23.65;
Found: C, 31.91; H, 3.89; N, 20.41; Br, 23.80.

## EXAMPLE 2

Preparation of 8-amino-9-(1,3-dihydroxy-2-propoxymethyl)guanine

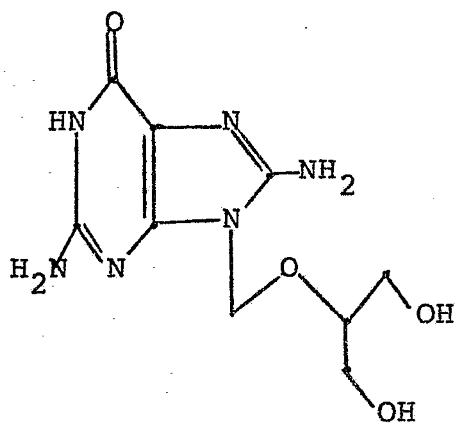

According to an adaptation of the method of Holmes and Robins, J. Am. Chem. Soc., 87, 1772 (1965), a mixture of 501 mg (1.5 mmole) of 8-bromo-9-(1,3-dihydroxy-2-propoxymethyl)guanine, 1.5 ml of hydrazine hydrate, and 45 ml of $H_2O$ was stirred under reflux for 2 days, by which time TLC (70:30:3 $CHCl_3$-MeOH-$H_2O$) indicated no remaining starting material. The solid which precipitated upon cooling was isolated on a filter. Recrystallization from $H_2O$ yielded 166 mg (41%) of cream-colored solid: m.p. $\nless$ 300°C. Satisfactory purity was confirmed by TLC (70:30:3 $CHCl_3$-MeOH-$H_2O$) and analytical reverse phase HPLC (Whatman $C_8$; 9:1 $H_2O$-MeOH), pmr (DMSO-$d_6$) $\delta$ 3.2-3.7 (m, 5H, $OCH(CH_2O)_2$), 4.64 (br t, 2H, OH), 5.28 (s, 2H, $NCH_2O$), 5.97 (s, 2H, 8-$NH_2$), 6.32 (s, 2H, 2-$NH_2$).

Anal. for $C_9H_{14}N_6O_4 \cdot 0.25\ H_2O$:
Calc'd: C, 39.34; H, 5.32; N, 30.59.
Found: C, 39.43; H, 5.38; N, 30.66.

## EXAMPLE 3

Preparation of 8-benzyloxy-9-(1,3-dihydroxy-2-propoxymethyl)guanine

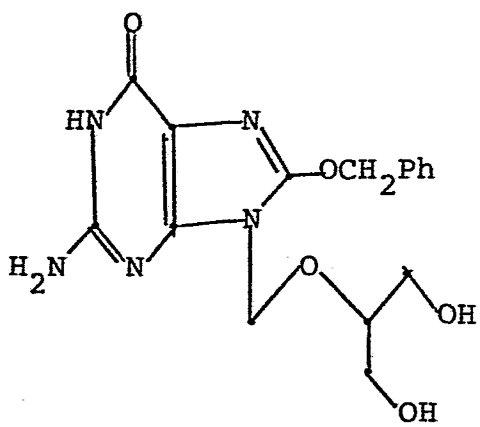

Clean sodium (207 mg, 9 mmole) was heated with an excess of benzyl alcohol (3.08 ml, 30 mmole) at 125°C under a nitrogen blanket. When all the sodium had reacted, the clear solution was cooled to 85°C, and with magnetic stirring, a solution of 8-bromo-9-(1,3-dihydroxy-2-propoxymethyl)guanine (1.000 g, 3 mmole) in sieve-dried dimethyl sulfoxide (5 ml) was added. The resulting clear yellow solution was maintained at 85°C with stirring under nitrogen for 16 hours, then cooled to 22°C and neutralized by adding acetic acid (0.60 ml).

The mixture was evaporated at 85°C/0.5 mm pressure leaving a sticky yellow solid (2.14 g) which was dissolved in warm aqueous 90% methanol (120 ml). Silica gel (10 g) was added and the mixture was again

evaporated at 85°C/0.5 mm pressure to dry powder, which was put on top of a 3 X 35 cm column of silica gel (100 g) prepared and developed in $CHCl_3$/aqueous 90% methanol in a ratio of 80/20. The desired product was eluted as single spot material. $R_f$ 0.65 (silica, $CHCl_3$/aq. 90% MeOH:70/30). Appropriate fractions were combined and evaporated to give an off-white solid (195 mg).

Recrystallization from aqueous 50% methanol (10 ml) gave colorless matted needles (120 mg), m.p. 221-226°, pmr ($d_6$-DMSO)$\delta$ 3.02-3.68 (m, 5H, $-CH(CH_2O-)_2$), 4.39 (t, 2H, J=6 Hz, $(-OH)_2$), 5.12 (s, 2H, $-NCH_2-$ or $-OCH_2Ar$), 5.27 (s, 2H, $-OCH_2Ar$ or $-NCH_2-$), 6.24 (s, 2H, $NH_2$); 7.18-7.36 (m, 5H, Ar).

EXAMPLE 4

Preparation of 9-(1,3-Dihydroxy-2-propoxymethyl)-8-hydroxyguanine

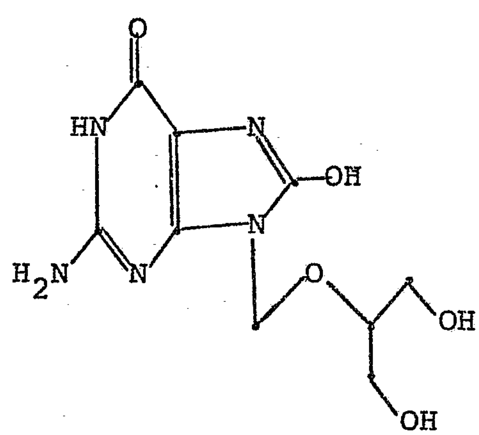

A suspension of 8-benzyloxy-9-(1,3-dihydroxy-2-propoxymethyl)guanine (50 mg) and 20% Pd $(OH)_2$-on-carbon catalyst (10 mg) in methanol (2 ml)

was vigorously stirred and hydrogenated at 23°C and atmospheric pressure for 30 minutes. Methanol (2 ml) and water (2 ml) were added and the mixture was heated to 70°C to dissolve the sparingly-soluble product. The hot mixture was then filtered through a 6 X 15 mm pad of 'Solkafloc' which was washed with more hot water (3 x 2 ml) and the combined filtrates were evaporated at 70°C/0.1 mm pressure. The colorless solid (35 mg) which was left was recrystallized from hot water (2 ml) to give colorless microprisms (16 mg), m.p. 280-285°C d. pmr ($D_2O$) $\delta$ 3.35 and 3.48 (d ABq, 4H, J=11.5, 6, and 5 Hz, $(-CH_2O-)_2$), 3.67 (quint., 1H, J=5 Hz, -O-CH), 5.17 (s, 2H, $-NCH_2O-$).

Anal. for $C_9H_{13}N_5O_5 \cdot 0.5\ H_2O$:
Calc'd: C,38.57; H, 5.03; N, 24.99;
Found: C, 38.50; H, 4.63; N, 24.77.

EXAMPLE 5

Preparation of 8-hydroxy-9-(4-hydroxybutyl)guanine

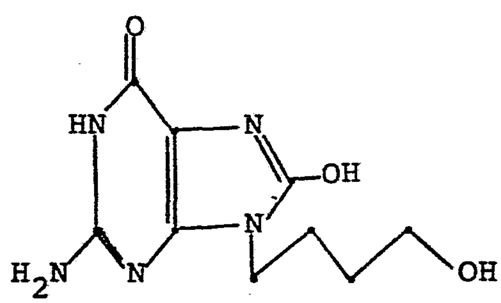

According to a modified general urea fusion method of L. F. Cavalieri and A. Bendich, J. Am. Chem. Soc., 72, 2587 (1950), a mixture of 6-(4-hydroxybutylamino)-5-nitrosoisocytosine* (2.73 g, 12.0 mmoles), 10% palladium-on-carbon (300 mg), and

2-methoxyethanol (150 ml) was shaken with hydrogen (initial pressure:approximately 3 atm) on a Parr apparatus until TLC [70:30:3 ($CHCl_3$-MeOH-$H_2O$)] indicated complete reaction (about 1 hour). The catalyst was removed by filtration under nitrogen, and the filtrate was concentrated to dryness under vacuum. To the residue was added 2.01 g (33.6 mmol) of urea, and the mixture was heated under $N_2$ in an oil bath at 160°C for 1.25 hour.

After cooling to approximately 0°C, the mixture was treated with cold water and stirred thoroughly. The resulting orange solid was collected on a filter. It was then dissolved as completely as possible in a small amount of 2.5 N NaOH, filtering to remove some dark insoluble matter. The filtrate was treated with charcoal and again filtered. Neutralization of the filtrate with AcOH produced precipitation. The solid was isolated on a filter and then recrystallized from water to yield 345 mg (12%) of white crystals: m.p.≮320°C; TLC: [70:30:3 ($CHCl_3$-MeOH-$H_2O$)]; 200 MHz pmr (DMSO-$d_6$) δ 1.2-1.8 (br m, 4H, $CH_2(C\underline{H}_2)_2CH_2$), 3.4 (m, 2H, $C\underline{H}_2OH$), 3.59 (t, J=7 Hz, 2H, $NC\underline{H}_2CH_2$), 4.41 (br t, 1H, OH), 6.50 (s, 2H, $NH_2$); pmr (DMSO-$d_6$) δ 10.6 (br s, 2H, $N^1H$, $N^7H$).

Anal. for $C_9H_{13}N_5O_3 \cdot 1.33\ H_2O$:
Calc'd: C, 41.06; H, 6.00; N, 26.60;
Found: C, 40.89; H, 5.77; N, 26.91.

---

* A. Yamazaki, Chem. Pharm. Bull., 17, 1268 (1969).

EXAMPLE 6

Preparation of 9-(4-Hydroxybutyl)-8-methylguanine

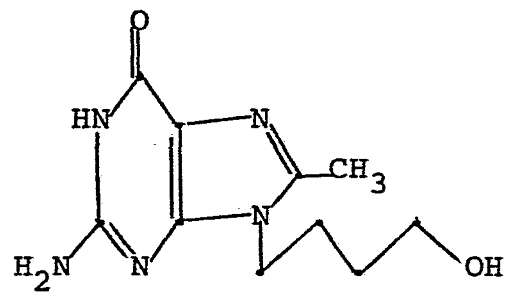

Step A: 5-Acetamido-6-(4-hydroxybutylamino)isocytosine

To a stirred mixture of 1.00 g (4.4 mmol) of 6-(4-hydroxybutylamino-5-nitrosoisocytosine*, 0.90 g (8.8 mmol) acetic anhydride, and 45 ml of AcOH was added 5.0 g of zinc dust. Stirring was continued at ambient temperature, an additional 2.0 g of zinc dust was added after 1 hour, and the reaction mixture was filtered after 2 hours. Concentration of the filtrate under high vacuum gave a solid residue, which was triturated with ethanol and then dried, yielding 652 mg (58%) of material suitable for use in the next step: m.p. 291-293°C dec; TLC [80:20:2 ($CHCl_3$-MeOH-$H_2O$)].

*A. Yamazaki, Chem. Pharm. Bull., 17, 1268 (1969).

Step B: 9-(4-Hydroxybutyl)-8-methylguanine

Using a modification of the general method of W. Pfleiderer and M. Shanshal, Liebigs Ann. Chem., 726, 20 (1969), a suspension of 5-acetamido-6-(4-hydroxybutylamino)isocytosine (500 mg, 2.0 mmol) in acetic anhydride (8 ml) was stirred at reflux under nitrogen for 66 hours. After concentration under

high vacuum, the solid residue was treated with 1N NaOH (20 ml) and the resulting solution was heated at reflux for 1 hour. The cooled solution was adjusted to pH 7 with AcOH and then concentrated to dryness. The residual solid was triturated and washed with water. Recrystallization from water yielded 47 mg (10%) of white crystals: m.p.≮325°C; TLC [80:20:2 ($CHCl_3$-MeOH-$H_2O$)]; pmr (DMSO-$d_6$) δ 1.1-1.9 (br m, 4H, $CH_2(C\underline{H}_2)_2CH_2$), 2.33 (s, 3H, $CH_3$), 3.39 (m, 2H, $C\underline{H}_2OH$), 3.89 (br t, J=7 Hz, 2H, $NCH_2C\underline{H}_2$), 6.57 (br s, 2H, $NH_2$); UV λ max (pH 1) 253 (ε 12,800), 278 (ε 8,520); λ max (pH 7) 250 (ε 13,700); λ max (pH 13) 268 (ε 5,860). Anal. ($C_{10}H_{15}N_5O_2 \cdot 0.25\ H_2O$) C, H, N.

Calc'd: C, 49.68; H, 6.46; N, 28.97.

Found: C, 49.94; H, 6.46; N, 29.23.

WHAT IS CLAIMED IS:

1. 8-Substituted-9-hydroxyalkyl- and hydroxyalkoxymethyl-guanines, having the formula:

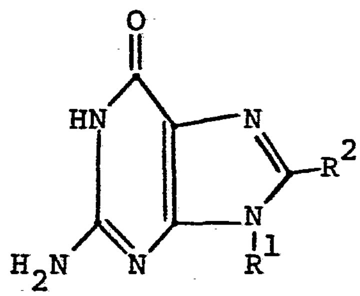

wherein:

$R^1$ is mono- or polyhydroxy-$C_1$-$C_6$-straight- or branched chain alkyl, containing 1 to 5 hydroxy groups, or mono- or polyhydroxy-$C_1$-$C_6$-straight-or branched-chain alkoxymethyl, containing 1 to 5 hydroxyl groups, not including $CH_2OCH_2CH_2OH$; and

$R^2$ is Cl, Br, F, I, $NH_2$, OH, SH, $R^3$, $SR^3$, $NHR^3$, $NR^3R^4$ or $OR^3$, where $R^3$ and $R^4$ are independently $C_1$-$C_{20}$ straight- or branched-chain, saturated or unsaturated alkyl, where the degree of unsaturation is limited to 3 double bonds; $C_1$-$C_{20}$ straight- or branched-chain, saturated or unsaturated alkyl, where the degree of unsaturation is limited to 3 double bonds, where said alkyl contains a $C_3$-$C_{10}$ mono- or bicyclic alkyl, aromatic or heteroaromatic; $C_3$-$C_{10}$ mono- or bicyclic, saturated or unsaturated alkyl, aromatic or heteroaromatic, where the degree of unsaturation

is limited to 5 double bonds; or where, in $NR^3R^4$, $R^3$ and $R^4$ together are a $C_3$-$C_{10}$ alkylene group;

or pharmaceutically-acceptable salts thereof.

2. An 8-substituted-9-hydroxyalkoxymethyl-guanine or pharmaceutically-acceptable salt thereof according to Claim 1, wherein $R^1$ is $CH_2OCH(CH_2OH)_2$ or $CH_2OCH_2CHOHCH_2OH$ and $R^2$ is $NH_2$.

3. A pharmaceutical composition comprising an amount of 8-substituted-9-hydroxyalkyl-guanine, 8-substituted-9-hydroxyalkoxymethyl-guanine or pharmaceutically-acceptable salt thereof according to Claim 1 effective to impart immunosuppresive activity, an antiviral effect, an antihyperuricemia effect or an anticancer effect, and a pharmaceutical carrier.

4. A pharmaceutical composition according to Claim 3, where one or more other active ingredient is also included with the composition.

5. A pharmaceutical composition according to Claim 3, wherein the amount is about 1 to about 250 mg.

6. Use of 8-substituted-9-hydroxyalkyl-guanine, 8-substituted-9-hydroxyalkoxymethyl-guanine, or pharmaceutically-acceptable salt thereof according to Claim 1, for the manufacture of a medicament for treating a virus infection, a cancer, a hyperuricemia or for imparting immunosuppresive activity in a mammalian or avian of piscine species.

0193454

Application number

European Patent Office

# EUROPEAN SEARCH REPORT

EP 86400334.8

| Category | DOCUMENTS CONSIDERED TO BE RELEVANT<br>Citation of document with indication, where appropriate, of relevant passages | Relevant to claim | CLASSIFICATION OF THE APPLICATION (Int. Cl 4) |
|---|---|---|---|
| P,X | EP - A1 - 0 145 207 (WARNER-LAMBERT COMPANY)<br>* Claims 1,3,4; example 3; page 3, lines 8-19 *<br>-- | 1-3,6 | C 07 D 473/18<br>A 61 K 31/52 |
| A | EP - A1 - 0 055 239 (ASTRA LAKE-MEDEL AKTIEBOLAG)<br>* Abstract *<br>-- | 1,3,6 | |
| A | AT - B - 352 751 (THE WELLCOME FOUNDATION LIMITED)<br>* Page 2, lines 1-17; example 1 *<br>-- | 1,3,6 | |
| D,A | BIOCHEMICAL PHARMACOLOGY, vol. 31, no. 2, 1982, Oxford, New York, Paris, Frankfurt<br>J.D. STOECKLER et al. "Inhibitors of purine nucleoside phosphorylase" pages 163-171<br>* Pages 164,167 *<br>-- | 1 | TECHNICAL FIELDS SEARCHED (Int. Cl 4)<br>C 07 D 473/00 |
| A | BIOCHEMICAL PHARMACOLOGY, vol. 30, no. 22, 1981, Oxford, New York, Frankfurt<br>P.M. KELLER et al. "Enzymatic phosphorylation of acyclic nucleoside analogs and correlations with anti-herpetic activities"<br>pages 3071-3077<br>* Page 3073, compound no. 22 *<br>---- | 1 | |

The present search report has been drawn up for all claims

| Place of search | Date of completion of the search | Examiner |
|---|---|---|
| VIENNA | 21-04-1986 | HERING |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document